# EUROPEAN PATENT APPLICATION

(11) **EP 1 690 512 A1**
(43) Date of publication of application: **16.08.2006**
(21) Application number: 06002670.5
(22) Date of filing: 09.02.2006
(51) Int. Cl.: A61F 2/06, A61F 2/84

(54) **Stent-graft delivery system and assembly method**

(30) Priority: 11.02.2005 US 56743
(71) Applicant: Medtronic Vascular, Inc., Santa Rosa, California 95403 (US)
(72) Inventor: Rust, Matthew, Santa Rosa, CA 95405 (US)
(74) Representative: Zimmermann, Gerd Heinrich

(57) **Abstract**

A system (100) for treating a vascular condition and methods for assembling such a system. The system (100) includes a catheter inner member (110), a stent framework (120), a graft material (130) aligned with the stent framework (120) to form a stent-graft assembly (150), and a slidable sheath (140). Multiple bands (115a,b,c,d,e) are spaced along the length of a distal portion (114) of the inner member (110), each band (115a,b,c,d,e) at least partially encircling the inner member (110). The stent framework (120) includes multiple rings (125a,b,c,d,e). The stent-graft assembly (150) is mounted on the inner member (110) such that the stent framework rings (125a,b,c,d,e) are interspaced with the inner member bands (115a,b,c,d,e). The movable sheath (140) encloses the stent-graft assembly (150). As the sheath (140) is withdrawn to deploy the stent-graft assembly (150), the interspaced bands (115a,b,c,d,e) and rings (125a,b,c,d,e) restrict axial movement of the assembly (150) and prevent longitudinal compression or buckling of the assembly (150), as anchoring of the stent-graft assembly (150) is distributed along the length of the inner member (110).

## Description

### TECHNICAL FIELD

This invention relates generally to biomedical systems for treating vascular conditions and to methods for manufacturing such biomedical systems. More specifically, the invention relates to a system that includes an increased friction inner member for stent-graft deployment and to methods for assembling such a system.

### BACKGROUND OF THE INVENTION

Stent-grafts are suitable for use in treating aneurysms. An aneurysm is a bulge or sac that forms in the wall of a blood vessel. If left untreated, the force of blood pressure in the aneurysm may cause the vessel to rupture. Aneurysms are often the result of fatty deposits on the vessel wall but may also result from other causes that weaken the vessel wall, including heredity, trauma, or disease.

During delivery of a self-expanding stent-graft, a sheath encloses the device and prevents expansion of the stent-graft from an unexpanded or crimped (compressed) configuration. The sheath is retracted to allow expansion of the stent-graft. Typically, a stop on the inner member prevents the proximal end of the stent-graft (the end nearest to the treating clinician) from moving past the stop as the sheath is retracted. However, the sheath retraction force may cause the stent-graft to attempt to move proximally, resulting in crumpling or buckling of the stent-graft when the proximal end of the device is held stationary by the stop. Such crumpling or buckling can result in a reduction of the effective length of the stent-graft and a discrepancy in the deployment location.

Similarly, during loading of the stent-graft into the sheath, a stent-graft without a longitudinal structural reinforcement can buckle or bunch up as the compressed stent-graft is drawn into the delivery sheath, potentially damaging the stent-graft and interfering with easy loading of the device into the sheath.

Therefore, it would be desirable to have an improved system for treating a vascular condition and methods for assembling such a treatment system that overcome the aforementioned and other disadvantages

### SUMMARY OF THE INVENTION

The above object is solved by a system for treating a vascular condition according to claim 1 and a method for assembling such a system according to claim 17. Further aspects, features, and details of the present invention as well as the advantages associated therewith are apparent from the dependent claims, the description and the accompanying drawings.

One aspect according to the present invention is a system for treating a vascular condition. The system comprises a catheter inner member, a stent framework, a graft material, and a movable sheath. A distal portion of the catheter inner member includes a plurality of bands spaced along the length of the distal portion, each band at least partially encircling the inner member. The stent framework includes a plurality of rings. The graft material is concentrically aligned with the stent framework to form a stent-graft assembly. The stent-graft assembly is mounted on the inner member such that the stent framework rings are interspaced with the inner member bands. The movable sheath encloses the stent-graft assembly and at least a portion of the inner member.

Another aspect according to the present invention is a method of assembling a system for treating a vascular condition. Multiple bands are attached to an outer surface of an inner member. The bands are distributed along the length of a distal portion of the inner member, each band at least partially encircling the inner member. A stent framework comprising a plurality of rings is concentrically aligned with a graft material to form a stent-graft assembly. The inner member is positioned within the stent-graft assembly such that the inner member bands are interspaced with the stent framework rings. The stent-graft assembly is radially compressed about the inner member. The stent-graft assembly and at least a portion of the inner member are positioned within a sheath.

The various aspects of the present invention can be advantageously applied in a method of treating a vascular condition. Therein, a sheathed self-expanding stent-graft assembly is delivered to a target region of a vessel via a catheter. The sheath is retracted from the stent-graft assembly while, at the same time, axial movement of spaced-apart stent framework segments of the stent-graft assembly is restricted at a plurality of spaced-apart stop regions on a distal portion of the catheter.

### BRIEF DESCRIPTION OF THE DRAWINGS

**FIG. 1** is an illustration of one embodiment of a system for treating a vascular condition, in accordance with the present invention;
**FIG. 2** is a cross-sectional view of the inner member of **FIG. 1;**
**FIG. 3** is a cross-sectional view of an alternative inner member in accordance with the present invention;
**FIG. 4** illustrates the system of **FIG. 1** at an intermediate stage of deployment of a stent-graft assembly at a treatment site;
**FIG. 5** is a flow diagram of one embodiment of a method of assembling a system for treating a vascular condition, in accordance with the present invention; and
**FIG. 6** is a flow diagram of an exemplary method of treating a vascular condition, utilizing a system in accordance with an embodiment of the present invention.

Like reference numbers are used throughout the drawings to refer to like elements.

### DETAILED DESCRIPTION

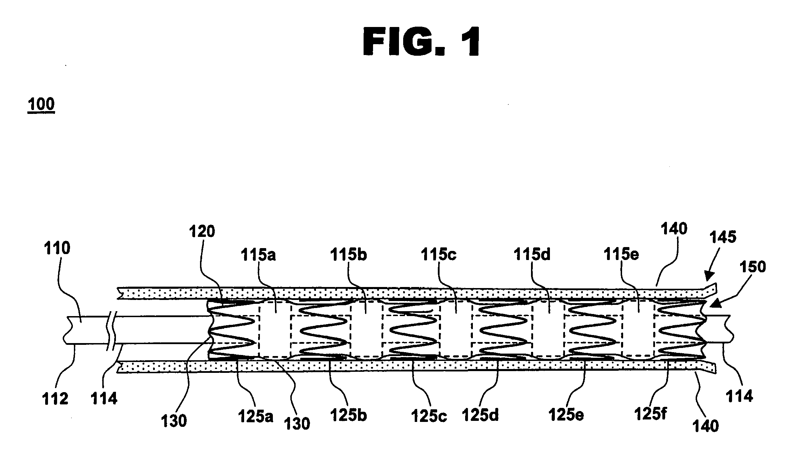

One aspect according to the present invention is a system for treating a vascular condition. One embodiment is illustrated at **100** in **FIG. 1.** The system comprises a catheter inner member **110,** a stent framework **120,** a graft material **130,** and a sheath **140.** Inner member **110** has a proximal portion **112** and a distal portion **114.** Distal portion **114** includes a plurality of bands **115a,b,c,d,e** spaced along its length. Stent framework **120** includes a plurality of rings **125a,b,c,d,e,f.** Stent framework **120** and graft material **130** combine to form a stent-graft assembly **150.** Sheath **140** is shown in cross-section to reveal inner member **110** and stent-graft assembly **150.** Bands **115a,b,c,d,e** and a portion of inner member **110** are shown in phantom within graft material **130.** Only a distal portion of system **100** is illustrated. As used herein, the terms "distal" and "proximal" are with reference to the treating clinician during deployment of the stent-graft assembly.

Inner member **110** is an elongated structure that, in the present embodiment, includes a central lumen through which a guidewire (not shown) may pass. Inner member **110** is formed using one or more biocompatible materials such as polyurethane, polyethylene, nylon, or polytetrafluoroethylene (PTFE). The proximal **112** and distal **114** portions of inner member **110** may be formed using the same or different materials. In the present embodiment, the two portions are formed from a continuous length of material. In another embodiment, the two portions may be formed separately and bonded one to the other. Forming the portions separately may provide cost savings and allows the two portions to have different characteristics; for example, it may be desirable for distal portion **114** to be stiffer than proximal portion **112** to ensure pushability of the inner member when delivering stent-graft assembly **150** to a treatment site. However, it should be understood that it is still within the scope of the present invention that the stiffness of the distal and proximal portions **114, 112** may be equal or even the proximal portion **112** may be stiffer than the distal portion **114.**

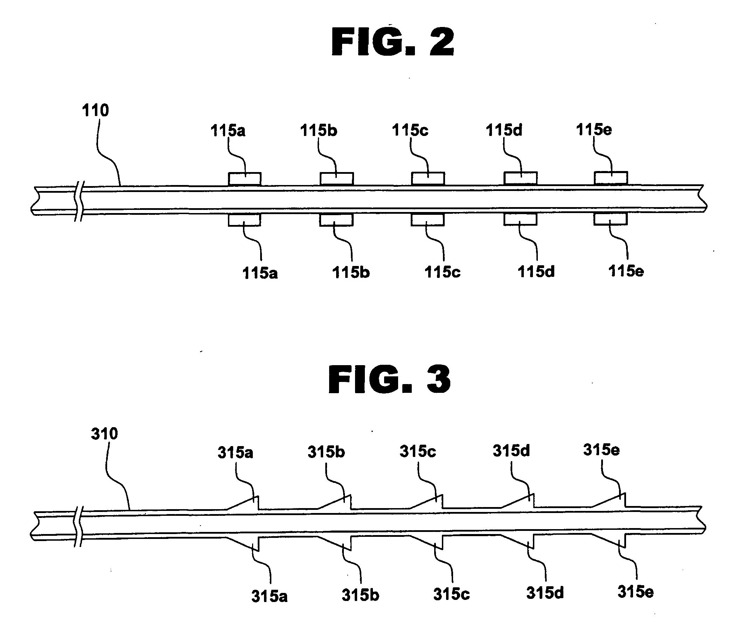

Inner member bands **115a,b,c,d,e** are spaced along the length of distal portion **114.** The bands may be evenly spaced along the length of distal portion **114** or the spacing may vary. The bands are shown in phantom in **FIG. 1** and in cross-section in **FIG. 2.** In the present embodiment, each band is continuous and fully encircles inner member **110.** In another embodiment, each band may partially encircle the inner member, leaving a gap between ends of the band. Alternatively, a number of individual band segments may be attached around the circumference of the inner member to form a segmented band encircling the inner member.

In the present embodiment, bands **115a,b,c,d,e** are cut from a length of biocompatible heat-shrink tubing and are frictionally attached to the outer surface of distal portion **114** by heat-shrink fitting. Other biocompatible materials may be used to form the bands, including, but not limited to, PEEK, a structural foams, a thermoplastic elastomer, a polyamide, a polymer, a ceramic, a metal, combinations thereof, and the like. At least one inner member band, e.g., **115,** may include a radiopaque marker to aid in positioning stent-graft assembly **150** at a treatment site. In another embodiment, one or more of the bands may be composed of a radiopaque material.

In alternative embodiments, the bands may be attached to the outer surface of the inner member by bonding methods known in the art. The bands may be bonded directly to the inner member or may be initially bonded to or formed as a part of a sleeve, which is bonded to the inner member. The bands may also be formed as integral parts of the inner member by molding the bands at the same time as the inner member, using the same or a different material from that comprising the inner member. For example, bands **315a,b,c,d,e** shown in **FIG. 3** are formed as integral parts of an alternative inner member **310.**

Inner member bands **115a,b,c,d,e** of system **100** are substantially rectangular in cross section, as can be seen in **FIG. 2.** The bands may also be substantially triangular in cross section, as seen in **FIG. 3.** One skilled in the art will appreciate that a wide range of cross-sectional configurations are possible. The inner member bands are sized longitudinally such that each band occupies the space between two stent framework rings. The thickness of the bands (i.e., the height of the bands above the adjacent inner member surface) may vary, with the thickness dependent upon the dimensions of stent-graft assembly **150** as well as the inner diameter of sheath **140.** As just one example, where a 21 French sheath is used, the thickness of an inner member band may be in the range of 0.1 millimeter to 3 millimeters.

Stent framework 120 is self-expanding from a radially compressed or crimped configuration and is formed from, for example, a nickel-titanium alloy, a nickel-cobalt alloy, a cobalt alloy, a thermoset plastic, stainless steel, a stainless steel alloy, a biocompatible shape-memory material, a biocompatible superelastic material, combinations of the above, and the like. In the present embodiment, stent framework rings 125a,b,c,d,e,f are fully detached one from another, with no connecting or longitudinal support members. If desired, bars or other structures may be added to connect all or some of the rings, thereby adding longitudinal column strength to the stent graft.

Graft material 130 is concentrically aligned with stent framework 120 to form stent-graft assembly 150. As illustrated in FIG. 1, graft material 130 is received within stent framework 120; however, the graft material may, instead, be positioned on the outside of the stent framework, enclosing rings **125a,b,c,d,e,f.** Graft material **130** may be formed from a biocompatible material such as polytetrafluoroethylene (PTFE), expanded polytetrafluoroethylene (ePTFE), spun polyester (PET), Dacron™, and the like. Graft material 130 may be attached to stent framework 120 using methods known in the art such as stitching or adhesive bonding. The dimensions of graft material 130 are dependent upon the dimensions of stent framework 120, with the thickness of the graft material determined at least in part by the intended use.

Stent-graft assembly 150 is mounted on inner member 110 by radially compressing stent-graft assembly 150 about inner member 110. The assembly is positioned on the inner member such that stent framework rings **125a,b,c,d,e,f** are interspaced with inner member bands 115a,b,c,d,e. As seen in FIG. 1, each inner member band is bordered by two stent framework rings (e.g., inner member band **115a** is bordered by stent framework rings **125a** and **125b).** In another embodiment, the number of inner member bands may equal or exceed the number of stent framework rings, with one or more bands positioned at one or both ends of the stent-graft assembly. In yet another embodiment, particularly one in which the stent framework rings are connected by support members, the rings and inner member bands may be interspaced with more than one stent framework ring positioned between two inner member bands.

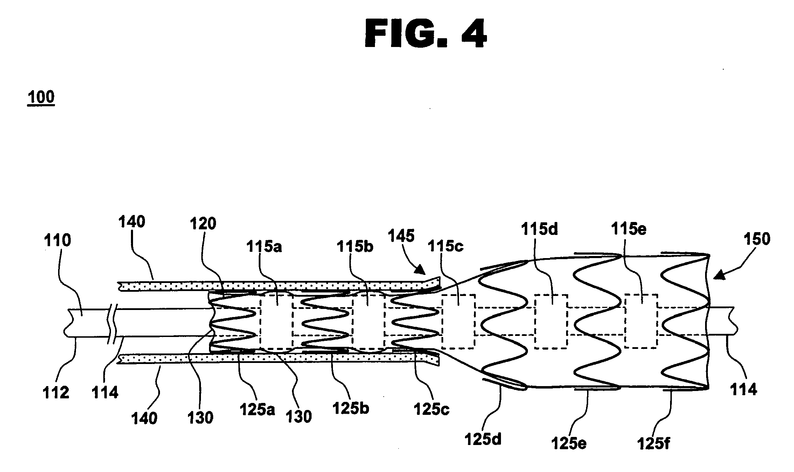

Sheath **140** (shown in cross-section in **FIGS. 1** and **4)** encloses stent-graft assembly **150** and a portion of inner member **110.** Sheath **140** is formed of one or more biocompatible materials and is sized such that it envelops the full length of the stent-graft assembly and maintains the stent-graft assembly in a compressed configuration about the inner member for delivery to a treatment site. Sheath **140** is movable with respect to stent-graft assembly **150** so that it may be retracted to allow expansion of the assembly. A distal portion of sheath **140** may be flared as seen in **FIGS. 1** and **4** at **145,** the flared portion improving the ease with which the stent-graft assembly may be loaded into the sheath during manufacture and also aiding in gradually transitioning each stent framework ring from a fully contracted configuration to an expanded configuration during deployment, as is illustrated in **FIG. 4.**

Depending on the size and configuration of the inner member bands and the stent framework rings, compressing the stent framework about the inner member may interlock the rings with the bands or, at a minimum, the inner member bands may provide increased friction to aid in retaining the compressed stent-graft assembly to the inner member.

In the present embodiment, when stent framework **120** is radially compressed about inner member **110,** inner member bands **115a,b,c,d,e** and stent framework rings **125a,b,c,d,e,f** are interlocked to prevent axial movement of stent-graft assembly **150** as sheath **140** is retracted. That is, each stent framework ring positioned by radial compression between two inner member bands (e.g., ring **125b** between bands **115a** and **115b)** is locked between the bands and restricted from axial movement in either a proximal or a distal direction. Sheath **140** prevents the stent framework rings from expanding radially outward and escaping the restriction of the inner member bands before the stent-graft is deployed.

As can be readily understood by referring to **FIG. 1,** the stent framework rings are restricted from axial movement in a proximal direction by the inner member band positioned proximal to the ring. For example, stent framework ring **125f** is prevented from axial movement in a proximal direction by inner member band **115e.** Thus, the stent framework ring at the distal end of the stent-graft assembly need not be locked between two inner member bands to prevent proximal displacement of the ring as the sheath is retracted. The stent framework ring at the proximal end of the stent graft assembly may be prevented from axial movement in a proximal direction by the anchoring effect of the bulk of the stent-graft assembly being already expanded and thereby anchored at the treatment site. Alternatively, an inner member band may be positioned at the proximal end of the stent-graft assembly, as previously noted, or a stop (not shown) may be included on the inner member.

The interlocking of inner member bands **115a,b,c,d,e** and stent framework rings **125a,b,c,d,e,f** also prevents stent-graft assembly **150** from prematurely escaping (i.e., jumping out) from the distal end of sheath **140,** providing a well-controlled delivery of the stent-graft. Thus, each stent framework ring is prevented from moving in a distal direction by the inner member ring positioned distal to the ring, with stent-graft assembly **150** being held in position until sheath **140** is drawn proximal to inner member ring **115a.**

In an embodiment in which the stent framework includes columnar support structures, the inner member bands may be positioned between some, but not all, of the stent framework rings, with the support structures preventing crumpling or buckling in areas of the stent framework not anchored by an inner member ring. In still another embodiment, the inner member bands may be distributed along the length of a distal portion of the inner member with no correlation between the spacing of the bands and the spacing of the stent framework rings. In this embodiment, the inner member bands produce a ribbed outer surface on the distal portion, with the ribbing providing increased friction to aid in anchoring the compressed stent-graft assembly to the inner member.

Deploying self-expanding stent-graft assembly **150** involves retracting sheath **140** proximally, while keeping inner member **110** and the attached stent-graft assembly **150** stationary at the treatment site. Forces acting on the stent-graft assembly during retraction of the sheath include the radial force of the sheath maintaining the assembly compressed about the inner member, combined with an axial (frictional) force resulting from the movement of the sheath over the outer surface of the stent-graft assembly. In a stent-graft assembly that is unrestrained along the length of the assembly, these forces may cause the distal end of the assembly to be drawn proximally along with the sheath, resulting in crumpling or buckling of the stent-graft assembly.

As explained above, in the present embodiment, the interspaced inner member bands **115a,b,c,d,e** and stent framework rings **125a,b,c,d,e,f** help anchor stent-graft assembly 150 to inner member 110 at multiple intervals along the length of the stent-graft assembly. As sheath 140 is withdrawn, the interspaced bands **115a,b,c,d,e** and rings **125a,b,c,d,e,f** distribute the deployment force to individual portions of the stent-graft assembly distal to an inner member band, thereby restricting axial movement of the assembly and preventing longitudinal compression or buckling of the assembly (train wrecking at an end). As sheath **140** is withdrawn, the exposed portion of stent-graft assembly **150** expands radially outward from inner member **110** as seen in **FIG. 4,** releasing the exposed assembly portion from the inner member.

In practice, these embodiments eliminate the need for the stent-graft assembly to include columnar support structures or for the stent framework to have column strength in order to be properly positioned at a treatment site. The embodiments also offer the advantage of allowing a stent-graft assembly to be positioned within its enclosing sheath by pulling on the inner member rather than by pushing on the assembly itself. Thus, a stent-graft assembly that does not have sufficient column strength or rigidity to be undamaged by the application of force to the distal end of the assembly may instead be pulled from the device by retracting the inner member. The inner member bands prevent axial movement of the stent framework rings as they are drawn into the sheath, just as they prevent axial movement of the rings as the sheath is retracted. The risk of longitudinal compression or buckling of the stent-graft during loading of the device into the sheath is eliminated, and ease of loading is improved.

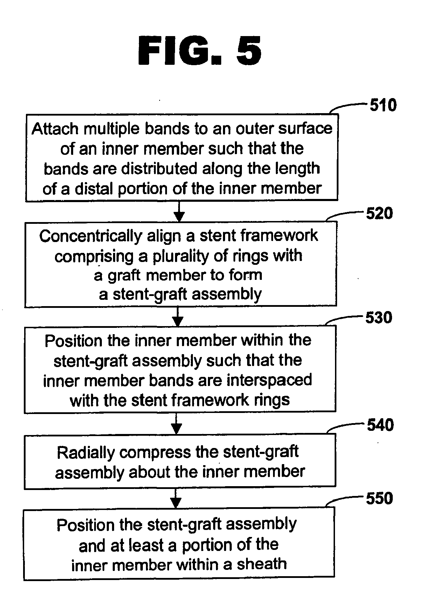

**FIG. 5** shows a flow diagram of a method in accordance with the present invention. Multiple bands are attached to an outer surface of an inner member **(Block 510).** The bands are distributed along the length of a distal portion of the inner member, each band at least partially encircling the inner member. In the present embodiment, the bands are cut from a length of heat-shrink tubing and are attached to the outer surface of the inner member by the application of heat. In another embodiment, individual bands or band segments may be attached to the inner member using other bonding methods known in the art. Alternatively, the bands may be first attached to, or formed as integral parts of, a sleeve that is attached to the outer surface of the inner member.

A stent framework comprising a plurality of rings is concentrically aligned with a graft material to form a stent-graft assembly **(Block 520).** In the present embodiment, the graft material is received within the stent framework. In another embodiment, the graft material may be positioned outside the stent framework. The graft material is attached to the stent framework using methods known in the art such as stitching or adhesive bonding.

The inner member is positioned within the stent-graft assembly such that the inner member bands are interspaced with the stent framework rings **(Block 530).** Alignment of the inner member bands relative to the stent framework rings may be accomplished visually.

The stent-graft assembly is radially compressed about the inner member **(Block 540).** The stent-graft assembly and at least a portion of the inner member are positioned within a sheath **(Block 550).** The inner member bands aid in retaining the stent-graft assembly to the inner member, allowing the assembly to be withdrawn from a radial compression device and positioned within the sheath by pulling on a proximal portion of the inner member rather than by pushing on one or both of the stent-graft assembly and the inner member. Thus, a stent-graft assembly that does not have sufficient column strength or rigidity to be undamaged by the application of force to the distal end of the assembly may instead be pulled from the device by retracting the inner member. The inner member bands prevent axial movement of the stent framework rings as they are drawn into the sheath, eliminating the risk of longitudinal compression or buckling of the stent-graft during loading of the device into the sheath, thereby improving ease of loading.

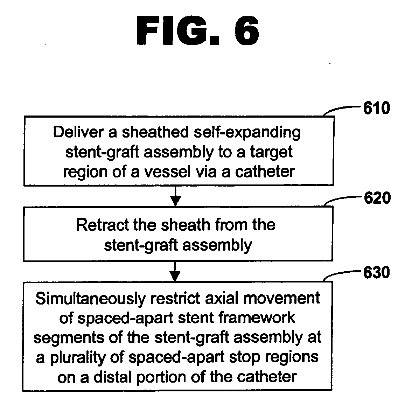

Next, a method of treating a vascular condition utilizing a system configured and assembled according to an embodiment of the present invention is described. **FIG. 6** shows a flow diagram of a method utilizing one embodiment in accordance with the present invention.

A sheathed self-expanding stent-graft assembly is delivered to a target region of a vessel via a catheter **(Block 610).** In the present example, the sheathed stent-graft assembly is as described above and illustrated in **FIG. 1.** The stent-graft assembly comprises a stent framework including a plurality of spaced-apart stent framework segments, shown in **FIG.** 1 as rings **125a,b,c,d,e,f.** The catheter includes an inner member such as that shown in **FIG. 1** at **110.** Inner member **110** includes a plurality of bands **115a,b,c,d,e** spaced along the length of distal portion **114** of inner member **110.**

The sheath is retracted from the stent-graft assembly (Block 620). At the same time, axial movement of the spaced-apart stent framework segments is restricted at a plurality of spaced-apart stop regions on the inner member (Block **630).** In the present example, inner member bands **115a,b,c,d,e** serve as the stop regions. The interlocked inner member bands and stent framework rings retain the stent-graft assembly to the inner member at multiple intervals along the length of the stent framework, thereby restricting axial movement of the stent-graft assembly during withdrawal of the sheath.

While various embodiments of the present invention are disclosed herein, changes and modifications can be made without departing from the spirit and scope of the invention.

## Claims

1. A system (100) for treating a vascular condition, comprising:
an inner member (110), a distal portion (114) of the inner member (110) including a plurality of bands (115a, b, c, d, e) spaced along the length of the distal portion (114), each band circumferentially aligned about the inner member (110);
a stent framework (120)including a plurality of rings (125a, b, c, d, e);
a graft material (130) concentrically aligned with the stent framework (120) to form a stent-graft assembly (150), the stent-graft assembly (150) mounted on the inner member (110) such that the stent framework rings (125a, b, c, d, e) are interspaced with the inner member bands (115a, b, c, d, e);
a sheath (140) slidably enclosing the stent-graft assembly (150) and at least a portion of the catheter inner member (110).

2. The system of claim 1 wherein each inner member band (115a, b, c, d, e) partially encircles the inner member (110).

3. The system of claim 1 wherein each inner member band (115a, b, c, d, e) fully encircles the inner member (110).

4. The system of claim 3 wherein each inner member band (115a, b, c, d, e) is continuous.

5. The system according to any of the preceding claims wherein the inner member bands comprise a thermoplastic elastomer.

6. The system of claim 5 wherein the inner member bands (115a, b, c, d, e) are lengths of heat-shrink tubing.

7. The system according to any of the preceding claims wherein the inner member bands (115a, b, c, d, e) are bonded to the inner member (110).

8. The system according to any of claims 1 to 6 wherein the inner member bands (315a, b, c, d, e) are integral to the inner member (310).

9. The system according to any of the preceding claims wherein each inner member band (115a, b, c, d, e) is substantially rectangular in cross section.

10. The system according to any of claims 1 to 8 wherein each inner member band (315a, b, c, d, e) is substantially triangular in cross section.

11. The system according to any of the preceding claims wherein at least a portion of at least one of the inner member bands (115a, b, c, d, e) is radiopaque.

12. The system according to any of the preceding claims wherein the stent framework rings (125a, b, c, d, e) are fully detached one from another.

13. The system according to any of the preceding claims wherein the stent framework (120) comprises a material selected from a group consisting of a nickel-titanium alloy, a nickel-cobalt alloy, a cobalt alloy, a thermoset plastic, stainless steel, a stainless steel alloy, a biocompatible shape-memory material, a biocompatible superelastic material, and any combination thereof.

14. The system according to any of the preceding claims wherein the graft material (130) is received within the stent framework (120).

15. The system according to any of the preceding claims wherein a distal portion (145) of the sheath (140) is flared.

16. The system according to any of the preceding claims wherein the distal portion (114) of the inner member (110) is stiffer than a proximal portion (112) of the inner member (110).

17. A method of assembling a system for treating a vascular condition, the method comprising:
attaching a plurality of bands to an outer surface of an inner member, the bands distributed along the length of a distal portion of the inner member, each band at least partially encircling the inner member;
concentrically aligning a stent framework with a graft material to form a stent-graft assembly, the stent framework comprising a plurality of rings;
positioning the inner member within the stent-graft assembly such that the inner member bands are interspaced with the stent framework rings;
radially compressing the stent-graft assembly about the inner member; and
positioning the stent-graft assembly and at least a portion of the inner member within a sheath.

18. The method of claim 17 further comprising cutting the bands from a length of heat-shrink tubing and wherein attaching a plurality of bands to the outer surface of the inner member comprises heating the bands to attach the bands to the outer surface of the inner member.

19. The method of claim 17 wherein attaching a plurality of bands to the outer surface of the inner member comprises attaching the bands to a sleeve and attaching the sleeve to the inner member.

20. The method of claim 17 further comprising forming the inner member bands as integral parts of a sleeve, and wherein attaching a plurality of bands to the outer surface of the inner member comprises attaching the sleeve to the inner member.

21. The method of any of claims 17 to 20 wherein positioning the stent-graft assembly within a sheath comprises pulling on a proximal portion of the inner member to position the stent-graft assembly and at least a portion of the inner member within the sheath.
